# EUROPEAN PATENT APPLICATION

(11) **EP 1 645 268 A1**
(43) Date of publication of application: **12.04.2006**
(21) Application number: 04746457.3
(22) Date of filing: 25.06.2004
(51) Int. Cl.: A61K 9/48, A61K 47/38, A61K 47/02

(54) **HARD CAPSULE**

(30) Priority: 27.06.2003 JP 2003184866
(71) Applicant: WAKUNAGA PHARMACEUTICAL CO., LTD., Osaka-shi, Osaka 532-0003 (JP)
(72) Inventor: MOTOUNE, Soko, c/o Wakunaga Pharmaceutical Co. Ltd, Akitakata-shi, Hiroshima 7391195 (JP); IKEDA, Yoichi, c/o Wakunaga Pharmaceutical Co. Ltd, Akitakata-shi, Hiroshima 7391195 (JP)
(74) Representative: Hartz, Nikolai
(86) International application number: PCT/JP2004/008988
(87) International publication number: WO 2005/000279

(57) **Abstract**

The present invention is directed to a hard capsule filled with a solution containing an active ingredient, **characterized in that** the solution contains an inorganic chloride and has a water content (w) of 10 < w ≤ 80% and a water activity (a) of 0.50 ≤ a ≤ 0.90, and that the capsule is made of a base material containing a cellulose derivative.

According to the present invention, a solution having high water content can be encapsulated, with its solution form being maintained. Thus, the present invention enables provision of a product which does not impair properties and stability of a drug or a similar substance, and does not impair sensation upon intake or eating sensation.

## Description

### Technical Field

The present invention relates to hard capsules useful as medical drugs, quasi-drugs, cosmetics, and foods, and more particularly to hard capsules which are able to hold ingredients of high water content.

### Background Art

In medical and food applications, capsules have conventionally been employed as the appliances required for improving compliance upon use. In particular, hard capsules can be produced easily and therefore have been widely used for containing solid substances such as powder, granules, and fine granules, or oily substances.

Hard capsules typically make use of gelatin as a shell-forming material, and therefore, they have high solubility in water. When an active component of high water content is directly charged in such a hard capsule, the internal solution renders the gelatin shells dissolved or moistened, permitting deformation of the shape of the capsules or leakage of the internal solution. As a result, the capsule has no value as a marketable product. Therefore, when the material to be encapsulated contains an aqueous solution, conventional approaches to avoid the effect of the water include addition of an excipient and solidification through, for example, concentration or drying, performed before encapsulation. In the case where a material is desired to be charged in capsules with its liquidity being maintained, the material is suspended or emulsified in an oily substance before being encapsulated. Alternatively, glycerol is added to the material and the resultant mixture is heated to evaporate the water so as to attain a water content of 10% or less, followed by filling in capsules formed of a cellulose derivative (e.g., Patent Document 1).

However, when subjected to a solidification process with heat, an animal or plant extract, such as a medicinal herb extract, is prone to change its intrinsic taste, odor, properties, and components. Moreover, when hard capsules containing such an extract are produced by means of solidification through addition of an excipient, formation of suspension or emulsification in an oily base, or reduction of a water content to 10% or less (which is attained by adding glycerol in an amount as high as 20% to 80% on a final product basis, followed by heating), drawbacks such as limitation being imposed on the amount of the active ingredient incorporated and a rise in production cost are unavoidable.

Meanwhile, soft capsules have been known to be able to contain liquid ingredients. In the case of soft capsules, encapsulation of ingredients of high water content, such as medicinal herb extracts, requires certain techniques. For example, a moisture-containing plant extract or a similar substance needs to be emulsified with a fatty acid glyceride to give a soft capsule (e.g., Patent Document 2), or, a gelatin sheet and a polysaccharide sheet need to be formed into a soft capsule having a layered structure in order to impart water resistance to a gelatin shell. (e.g., Patent Document 3). As a result, its production steps and production conditions get intricate, raising concern about increased cost.

Therefore, there has been a need for a technique capable of, through simple production steps and at low cost, encapsulating an aqueous extract of medicinal herbs, an aqueous extract of animal or plant origin, or a similar substance without permitting volatilization or degradation of active ingredients, while the resultant capsule products do not impair sensation (taste, odor, etc.) upon intake.
Patent Document 1: U.S. Patent No. 6,238,696
Patent Document 2: JP-A-52-35178
Patent Document 3: JP-A-63-164858

### Disclosure of the Invention

An object of the present invention is to provide a hard capsule filled with a solution containing an active ingredient of high water content, such as an aqueous extract of medicinal herbs, an aqueous extract of animal or plant origin, or a similar substance, without impairing its quality for a prolonged period of time.

In view of the foregoing, the present inventors have performed extensive studies on the relation between properties of a base material of a capsule and water content of the solution to be encapsulated, and have found that, through use in combination of a solution containing an active ingredient and an inorganic chloride and having a water content and a water activity each falling within a specific range and capsules formed of a specific capsule base material, the solution can be encapsulated in the capsules with its solution form being maintained, whereby hard capsules filled with the solution which can be stored for a long period of time can be prepared. The present invention has been accomplished on the basis of this finding.

Accordingly, the present invention provides a hard capsule filled with a solution containing an active ingredient, characterized in that the solution contains an inorganic chloride and has a water content (w) of 10 < w ≤ 80% and a water activity (a) of 0.50 ≤ a ≤ 0.90, and the capsule is made of a base material containing a cellulose derivative.

According to the present invention, a solution containing an active ingredient and having high water content can be encapsulated without use of an excipient or a similar additive, with its solution form being maintained. The capsules containing the solution are stable and do not undergo wetting or deformation during a long-term storage. Accordingly, the present invention enables provision of a product which does not impair properties and stability of a drug or a similar substance in the presence of a solution having a high water content, and does not impair sensation upon intake or texture. The hard capsules of the present invention can be produced efficiently, because their contents can be readily charged into the capsule shells owing to their liquid nature and the volume of the contents are easily adjusted.

### Best Mode for Carrying Out the Invention

The solution to be enclosed in the hard capsule of the present invention (hereinafter referred to as "internal solution") is a solution containing an inorganic chloride in addition to an active ingredient, and has a water content (w) of 10 < w ≤ 80% and a water activity (a) of 0.50 ≤ a ≤ 0.90.

As used herein, the term "water activity (a)" of a water-containing medium is defined as the ratio P/Po, of water vapor pressure of the medium (P) to vapor pressure of pure water (P₀) under the conditions where both pressures are measured at the same temperature. The water activity is a parameter representing activity of water in a medium.

No particular limitation is imposed on the internal solution so long as the solution has a water content (w) falling within a range of 10 < w ≤ 80% and a water activity (a) falling within a range of 0.50 ≤ a ≤ 0.90. The internal solution is not limited to aqueous solutions. When a portion of active ingredients is difficult to dissolve in water, a suspension or an O/W-type emulsion may be employed.

No particular limitation is imposed on the active ingredients of the internal solution, and they may be compounds or compositions of arbitrary substances, such as medical drugs, natural materials, foods, herbal medicines in the form of extracts, plant extracts, and fermented matter.
Specifically, examples of the active ingredients include aqueous extracts prepared by subjecting medicinal herbs, animal- or plant-originating substances, fermented matter, or like substances to extraction with water, hydrated alcohol, or a similar solvent. Specific examples falling under this category include herbal medicines in the form of aqueous extracts, such as ginseng, garlic, Acanthopanax senticosus Harms, Angelica sinensis, Rehmammia glutinosa, Citrus reticulata, Cuscuta chinensis, Schizandra chinensis, and Ophiopogon japonicus; aqueous extracts of herbal mixtures employed in kanpo (traditional Chinese medicine), available under the names of kakkon-to, bakumonto-to, sho seiryu-to, orengedoku-to, shimotsu-to, and shakuyaku kanzo-to; aqueous extracts of animal- or plant-origin, prepared from, for example, blueberries, green tea leaves, herbs, mushrooms, and mamushi (Japanese copperhead); and aqueous extracts of fermented matter produced by fermenting grains, plants, or marine products with microorganisms such as koji mold, beni koji mold, lactic acid bacteria, acetic acid bacteria, Bacillus natto, and yeast. Other examples of the internal solution include solutions in water of aqueous vitamins such as vitamin B1, vitamin B2, niacin, vitamin B6, vitamin B12, vitamin C, pantothenic acid, biotin, folic acid, and pantothenyl alcohol; solutions in water of dextromethorphan hydrobromide, acetaminophen, chlorphenylamine maleate, potassium guaiacolsulfonate, caffeine, dihydrocodeine phosphate, methylephedrine hydrochloride, and water-soluble azulene; and suspensions of aldioxa, magnesium hydroxide, sucralfate, magnesium aluminometasilicate, and synthetic hydrotalcite. In particular, herbal medicines in the form of aqueous extracts, aqueous extracts of animal- or plant-origin, and aqueous extracts of fermented matter are preferred, because they are stable as aqueous solutions, and they can be handled in their solution form during the capsule formulation process.

According to the present invention, an inorganic chloride is incorporated into the internal solution. By virtue of co-presence of the inorganic chloride, a solution of high water content can be encapsulated in the capsules with its solution form being maintained, whereby capsules filled with a solution which can be stored for a long period of time can be produced.
Examples of the inorganic chloride include inorganic chlorides which releases chloride ions in a solution, such as potassium chloride, sodium chloride, calcium chloride, magnesium chloride, ammonium chloride, and barium chloride. Among them, sodium chloride, calcium chloride, and magnesium chloride are preferred. These inorganic chlorides may be used singly or in combination of two or more species.
The inorganic chloride is incorporated into an internal solution in an amount of 0.01 to 0.45 g/mL, preferably 0.20 to 0.45 g/mL, in order to enhance stability of capsules.

If needed, the internal solution may contain other ingredients, such as sweetener, acidulant, stabilizer, thickener, pH regulator, preservative, colorant, and flavoring agent, which are generally used in the production of foods and drugs.

Here, examples of the sweetening agent include sugars such as sucrose, lactose, fructose, and glucose; sugar alcohols such as sorbitol, erythritol, mannitol, xylitol, and trehalose: glycyrrhizin; aspartame; and stevia. These sweetening agents may be used singly or in combination of two or more species.

As the acidulant, those commonly employed in the production of drugs and foods may be used. Examples of acidulants include citric acid, malic acid, succinic acid, fumaric acid, tartaric acid, and lactic acid. These acidulants may be used singly or in combination of two or more species.

Examples of the stabilizer include antioxidants such as ascorbic acid, erythorbic acid, and sodium pyrosulfite; dispersants such as sodium pyrophosphate, sodium polyphosphate, and sodium metaphosphate; surfactants such as sucrose fatty acid esters, polyoxyethylene hydrogenated castor oil, and polyoxyethylene polyoxypropylene glycol; cyclodextrins such as cyclodextrin, glucosyl-cyclodextrin, maltosyl-cyclodextrin, and hydroxypropyl-cyclodextrin. These stabilizers may be used singly or in combination of two or more species.

Examples of the thickener include polymers such as dextrin, sodium alginate, propylene glycol alginate, tragacanth powder, xanthan gum, carrageenan, agar, pectin, carboxymethylcellulose-Na, hydroxypropyl cellolose, polyvinyl alcohol, and polyvinyl pyrrolidone. These thickeners may be used singly or in combination of two or more species.

As for the pH regulator, those generally employed in the shape-imparting art may be used. Examples thereof include organic and inorganic acids such as hydrochloric acid, acetic acid, phosphoric acid, citric acid, malic acid, succinic acid, fumaric acid, tartaric acid, lactic acid, and salts of any of these acids, alkalis such as sodium hydroxide, potassium hydroxide, sodium hydrogencarbonate, and sodium dihydrogenphosphate. These pH regulators may be used singly or in combination with one or more of them.

Examples of the preservative include benzoic acids, sorbic acids, p-hydroxybenzoic esters, and salicylic acids. These preservatives may be used singly or in combination of two or more species.

Examples of the colorant include caramel, sodium copper chlorophyllin, riboflavin sodium phosphate, indigocarmine, Brilliant Blue, tartrazine, sunset yellow, new coccine, amaranth, and erythrosine. These colorants may be used singly or in combination of two or more species.

As for the flavoring agents, those generally employed for the manufacture of drugs and foods may be used, including, for example, fennel oil, orange oil, cinnamon oil, orange peel oil, mentha oil, vanillin, and eucalyptus oil. In addition to these, there may be employed natural-originating flavors and compounded flavors which are produced using any of these materials as raw material.

The foregoing internal solutions may be adjusted so as to be 10 < w ≤ 80% in the water content (w) and 0.50 ≤ a ≤ 0.90 in the water activity (a), respectively. Preferably, the water content is 10 < w ≤ 75% and the water activity is 0.50 ≤ a ≤ 0.80, with 10 < w ≤ 70% and 0.50 ≤ a ≤ 0.75 being more preferred.

When the water content of the internal solution exceeds 80% or the water activity exceeds 0.90, stability of the capsules holding the solution is reduced, which is not preferred. Meanwhile, a water content less than 10% or a water activity below 0.50 should also be avoided, because under such conditions, the viscosity of the internal solution extremely increases, thereby failing to attain smooth flow of the solution, so that it becomes difficult to fill the shells during production of capsules.

No particular limitation is imposed on the methods of adjusting water content (w) or water activity (a). For example, either of the following 1) or 2) may be performed: 1) Soluble solid matter is caused to dissolve in cold water or lukewarm water until a target water content and a target water activity are achieved, 2) in the case where an animal-or plant-derived crude drug in the aqueous extract form is employed, the relevant animal or plant material is extracted with water or a water-ethanol mixture, and the resultant filtrate is concentrated with the application of heat (25 to 50°C) under reduced pressure until a target water content and a target water activity are achieved.

The capsule shells of the hard capsules according to the present invention are made of a base material containing a cellulose derivative.

Examples of the cellulose derivative include hydroxypropyl methylcellulose, methylcellulose, hydroxypropyl cellulose, hydroxyethyl cellulose, hydroxypropyl methylcellulose phthalate, hydroxypropyl methylcellulose acetate succinate, carmelose, carboxymethylethyl cellulose, cellulose acetate phthalate, and ethylcellulose. In particular, hydroxypropyl methylcellulose and methylcellulose are preferred. The cellulose derivatives may be used singly or in combination of two or more species.

The cellulose derivative is incorporated in an amount of 15 to 30% by weight on the basis of the base material solution employed during the production of capsules. Also, additives which are generally employed in the shape-imparting art, such as a gelling agent, a gelling aid, a colorant, a plasticizer, an emulsifier, a dispersant, and a preservative may be added to the capsule base material.

Examples of the gelling agent to be employed include carrageenan, xanthan gum, locust bean gum, gellan gum, gum arabic, guar gum, tamarind-seed-derived polysaccharides, pectin, curdlan, gelatin, furcellaran, and agar. Preferably, carrageenan is employed. These materials may be used singly or in combination of two or more species. The amount of the gelling agent to be added is 0.1 to 1.0% by weight on the basis of the base material solution employed for the production of capsules.

Examples of the gelling aid to be employed include water-soluble compounds which release calcium ions, potassium ions, ammonium ions, sodium ions, or magnesium ions, and specific examples include calcium chloride, potassium chloride, ammonium chloride, ammonium acetate, potassium phosphate, potassium citrate, sodium chloride, magnesium sulfate, and organic acids and water soluble salts thereof (for example, citric acid or sodium citrate). Water-soluble compounds providing potassium ions or ammonium ions are preferably employed. The amount of the gelling aid to be added is 0.01 to 1.0% by weight on the basis of the base material solution employed for producing the capsules.

When a colorant is incorporated, any of ordinarily available additives in the production of capsules may be used in suitable amounts. Examples of the colorant include caramel, sodium copper chlorophyllin, riboflavin sodium phosphate, indigocarmine, Brilliant Blue, tartrazine, sunset yellow, new coccine, amaranth, erythrosine, titanium oxides, and iron oxides. These colorants may be used singly or in combination of two or more species.

Examples of the plasticizer include triethyl citrate, triacetin, glycerol, D-sorbitol, polyethylene glycol, acetylated monoglyceride, organic acids (e.g., citric acid, malic acid, and lactic acid), calcium carbonate, and surfactants, and these plasticizers may be used singly or in combination of two or more species.

The hard capsules according to the present invention can be produced as follows. As described above, the water content (w) and the water activity (a) of an internal solution are adjusted to 10 < w ≤ 80% and 0.50 ≤ a ≤ 0.90, respectively, and the resultant solution is charged into the aforementioned capsules by use of a conventional machine for filling capsules with a liquid-form drug designed to fill capsules with oily substances. Thereafter, in each capsule, the joint portion of the capsule body and its corresponding cap is sealed with an aqueous ethanol solution of a cellulose derivative, followed by drying in air.

Moreover, if necessary, the hard capsules of the present invention may be coated with a coating agent, or with two or more coating agents in combination, containing, for example, hydroxypropyl methylcellulose phthalate, hydroxypropyl methylcellulose acetate succinate, carboxymethylethyl cellulose, cellulose acetate phthalate, polyvinylacetal diethylaminoacetate, or a methacrylate copolymer.

Packaging of the thus-produced capsules may be performed in any conventional mode, by the use of, for example, bottles, aluminum foil, PTPs (press through packages), etc.

Preferred examples of the hard capsule of the present invention include those having a property that the internal solution is a medicinal herb extract having a water content (w) of 30 ≤ w ≤ 50% and a water activity (a) of 0.60 ≤ a ≤ 0.80, and the capsule shell contains hydroxypropyl methylcellulose, a gelling agent, and a gelling aid. In particular, a capsule which is #2 to #00 in capsule size and packed in bottles or aluminum pouches is preferred.

The present invention will next be described in more detail by way of examples, which should not be construed as limiting the invention thereto.

### Example 1

An aqueous sodium chloride solution containing a ginseng extract was prepared so that the water content was 74% or 78% and the water activity was 0.80 or 0.83. The solution was placed in #0 capsules made of hydroxypropyl methylcellulose, and the joining portion of the capsule body and its corresponding cap was sealed with an aqueous ethanol solution of hydroxypropyl methylcellulose, followed by drying in air. The resultant capsule samples were put in glass bottles, and stored at 40°C for one month. Whether there had been any leakage of the internal solution from the hard capsules was visually checked. The results are shown in Table 1. Neither leakage of internal solution from the hard capsules nor change of the capsule shape was observed.

**Table 1**

| | | |
|---|---|---|
| Water content (%) | 74 | 78 |
| Water activity | 0.80 | 0.83 |
| Sodium chloride concentration (g/mL) | 0.30 | 0.26 |
| 40°C, one month | No liquid leakage | No liquid leakage |

### Example 2

An aqueous calcium chloride solution containing a ginseng extract was prepared so that the water content was 67% or 70% and the water activity was 0.75 or 0.78. The solution was placed in #0 capsules made of hydroxypropyl methylcellulose. Subsequently, the capsule was sealed in a manner similar to that employed in Example 1, followed by drying in air. The resultant capsule samples were put in glass bottles, and stored at 40°C for one month. Whether there had been any leakage of the internal solution from the hard capsules was visually checked. The results are shown in Table 2. Neither leakage of internal solution from the hard capsules nor change of the capsule shape was observed.

**Table 2**

| | | |
|---|---|---|
| Water content (%) | 67 | 70 |
| Water activity | 0.75 | 0.78 |
| Calcium chloride concentration (g/mL) | 0.41 | 0.37 |
| 40°C, one month 40°C, one month | No liquid leakage | No liquid leakage |

### Example 3

An aqueous magnesium chloride solution containing a ginseng extract was prepared so that the water content was 66% or 70% and the water activity was 0.53 or 0.60. The solution was placed in #0 capsules made of hydroxypropyl methylcellulose. Subsequently, the capsule was sealed in a manner similar to that employed in Example 1, followed by drying in air. The resultant capsule samples were put in glass bottles, and stored at 40°C for one month. Whether there had been any leakage of the internal solution from the hard capsules was visually checked. The results are shown in Table 3. Neither leakage of internal solution from the hard capsules nor change of the capsule shape was observed.

**Table 3**

| | | |
|---|---|---|
| Water content (%) | 66 | 70 |
| Water activity | 0.53 | 0.60 |
| Magnesium chloride concentration (g/mL) | 0.45 | 0.40 |
| 40°C, one month | No liquid leakage | No liquid leakage |

### Comparative Example 1

An aqueous fructose glucose solution containing a ginseng extract was prepared so that the water content was 40% or 50% and the water activity was 0.85 or 0.88. The solution was placed in #0 capsules made of hydroxypropyl methylcellulose. Subsequently, the capsule was sealed in a manner similar to that employed in Example 1, followed by drying in air. The resultant capsule samples were put in glass bottles, and stored at 40°C for one month. Whether there had been any leakage of the internal solution from the hard capsules was visually checked. The results are shown in Table 4. In all samples, liquid leakage of internal solution from the hard capsule was observed after storage for one week.

**Table 4**

| | | |
|---|---|---|
| Water content (%) | 40 | 50 |
| Water activity | 0.85 | 0.88 |
| 40°C, one month | liquid leakage | liquid leakage |

## Claims

1. A hard capsule filled with a solution containing an active ingredient, **characterized in that** the solution contains an inorganic chloride and has a water content (w) of 10 < w ≤ 80% and a water activity (a) of 0.50 ≤ a ≤ 0.90, and that the capsule is made of a base material containing a cellulose derivative.

2. A hard capsule as described in claim 1, wherein the cellulose derivative is one or more members selected from the group consisting of hydroxypropyl methylcellulose, methylcellulose, hydroxypropyl cellulose, and hydroxyethyl cellulose.

3. A hard capsule as described in claim 1 or 2, wherein the inorganic chloride is one or more members selected from the group consisting of sodium chloride, magnesium chloride, and calcium chloride.

4. A hard capsule as described in any one of claims 1 to 3, wherein the active ingredient is selected from the group consisting of an aqueous extract of a medicinal herb, an aqueous extract of animal- or plant-origin, and an aqueous extract of fermented matter.
